Europäisches Patentamt

(19) European Patent Office

Office européen des brevets

(11) Publication number: **0 082 894**
**B1**

(12) **EUROPEAN PATENT SPECIFICATION**

(45) Date of publication of patent specification: **13.08.86**

(21) Application number: **81201409.0**

(22) Date of filing: **30.12.81**

(51) Int. Cl.⁴: **A 61 F 5/47**

(54) **Method of making intrauterine devices.**

(43) Date of publication of application:
**06.07.83 Bulletin 83/27**

(45) Publication of the grant of the patent:
**13.08.86 Bulletin 86/33**

(84) Designated Contracting States:
**AT BE CH DE FR GB IT LI LU NL SE**

(56) References cited:
**WO-A-81/00356**
**US-A-3 807 395**
**US-A-3 898 986**
**US-A-3 913 573**
**US-A-3 993 058**
**US-A-4 188 951**

(73) Proprietor: **THE POPULATION COUNCIL, INC.**
**One Dag Hammarskjöld Plaza**
**New York New York 10017 (US)**

(72) Inventor: **Robertson, Dale N.**
**5 Tempe Court**
**Scotch Plains New Jersey 07076 (US)**
Inventor: **Braun, John**
**128 Carle Road**
**Westbury New York 11590 (US)**

(74) Representative: **van der Beek, George Frans**
**et al**
**Nederlandsch Octrooibureau Johan de Wittlaan**
**15 P.O. Box 29720**
**NL-2502 LS 's-Gravenhage (NL)**

## Description

The invention relates to a method of making a combination of intrauterine device and drug release element comprising providing a formable silicone rubber as a carrier material and providing a non-medicated and relatively stable platform for intrauterine placement.

Such a method is known from US—A—3,913,573. An intrauterine device (IUD) provides a system for timed released, local administration of drug. According to this known method the stable platform is provided with a cavity for arranging certain types of drugs in a solid or liquid medium, which drugs are released gradually into the vagina over a prolonged period of time. The drug release rate is controlled by the drug permeable walls of the device.

The drug can be incorporated in an element, which may be arranged in the cavity within the platform.

The drug may also be arranged within a hollow tube of silicone rubber.

Normally room temperature vulcanizing silicone rubbers are used.

Because room temperature vulcanizing (RTV) silicone rubbers have been found to lack strength necessary in many applications, fillers are added for reinforcement. The fillers in RTV silicones have been determined to be undesirable in drug release articles, however. The fillers, it is said, adversely affect the release of the drug. For this reason, the low temperature vulcanizing (RTV) silicone rubbers have been recommended for the base or carrier material in drug release devices.

The method according to the present invention is characterized by mixing together a drug for in utero administration and the formable carrier material, the rubber of which being a non-filler-containing silicone rubber, forming the mixture of drug and the carrier material into an attachment adapted to fit onto the platform, and mounting the attachment on the platform for controlled release of the drug in utero, said mounting being effected by expanding the attachment by immersion in a solvent suitable for swelling the rubber and in which the rubber and the drug are substantially insoluble, and then shrinking the expanded attachment about a portion of the platform.

According to this invention, an intrauterine controlled release drug delivery article is associated with a proven IUD platform. The drug release article is constructed for attachment to the platform, which is nonmedicated and relatively stable and can be of a proven design. The attachment can be molded sleeve tightly secured on a stem of an extant IUD.

In a preferred embodiment, an outer drug permeable tube surrounds the sleeve, controlling the rate of administration of the drug contained in the sleeve. RTV (Room Temperature Vulcanisable) silicone rubbers can be employed as the base or carrier material of the sleeve. Removal of the fillers from commercially available silicone rubber, for example by centrifuging, makes the silicone rubber more satisfactory for drug release. The use of the outer tube in tightly fitting relation over the sleeve can impart structural integrity to the fillerless silicone rubber of the attachment to permit it to be handled in an ordinary way prior to insertion without deterioration.

The method of manufacturing the attachment and securing it on the extant IUD provides an easy to use series of steps wherein multiple attachments can be molded and securely fitted onto IUD stems. There is no need to specially manufacture the platform or to adapt it specially for drug release. Silicone rubber, such as the aforementioned fillerless RTV silicone, can be mixed with the drug and molded in a multicavity mold to form the sleeves. The sleeves are then swollen in a solvent, and slipped onto the IUD stem. There they contract tightly about the IUD stem.

The process can include the steps of centrifuging commercially available RTV silicone rubber to remove the fillers and placing over the sleeve a drug permeable covering such as the above-mentioned length of silicone rubber tubing.

The outer tubing that insures structural integrity of the fillerless RTV, while helping to control the dosage, can, likewise, be swollen in a solvent, slipped onto the drug-containing sleeve, and permitted to shrink tightly thereabout. With the sleeve and tubing tightly fitted onto the stem, the attachment will withstand normal handling that would otherwise be impermissible for fillerless RTV silicone rubber articles.

Because the drug release sleeves can be applied to known nonmedicated, and relatively stable IUD configurations, no modification of a physician's IUD insertion procedure is necessary. "Relatively stable", as used herein, means that the IUD or platform to which the sleeve is attached is not subject to rapid deterioration or bioerosion *in utero*, but provides a long-lasting device retaining the medicated sleeve. The closed platform can be a commercially available IUD of known contraceptive effect. The physician can continue to employ the IUD configuration of his choice. The IUD manufacturer need make no modification in the IUD to accommodate or incorporate the drug release article. Dosage or rate of release of drug is a function of the dimensions of the sleeve, and the thickness of the outer covering tube, as well as the amount of drug mixed into the silicone rubber carrier material. Selecting or modifying dosage, then, requires no change in the platform structure.

The foregoing and other advantages of the invention will appear more fully in relation to the following detailed description of a preferred embodiment of the invention, as illustrated in the drawings.

Figure 1 is an enlarged plan view, partially in seciton, and illustrates an IUD platform carrying a drug-dispensing attachment.

Figure 2 is a diagrammatic illustration of the steps in the process of making the combination IUD and attachment of Figure 1.

Figure 3 is an enlarged side elevational view of mold and syringe combination for making drug dispensing sleeves to be mounted on existing IUD's.

Figure 4 is a top plan view of one multicavity plate that forms a part of the mold of Figure 3.

Figure 5 is a partially sectional view of the syringe of Figure 3.

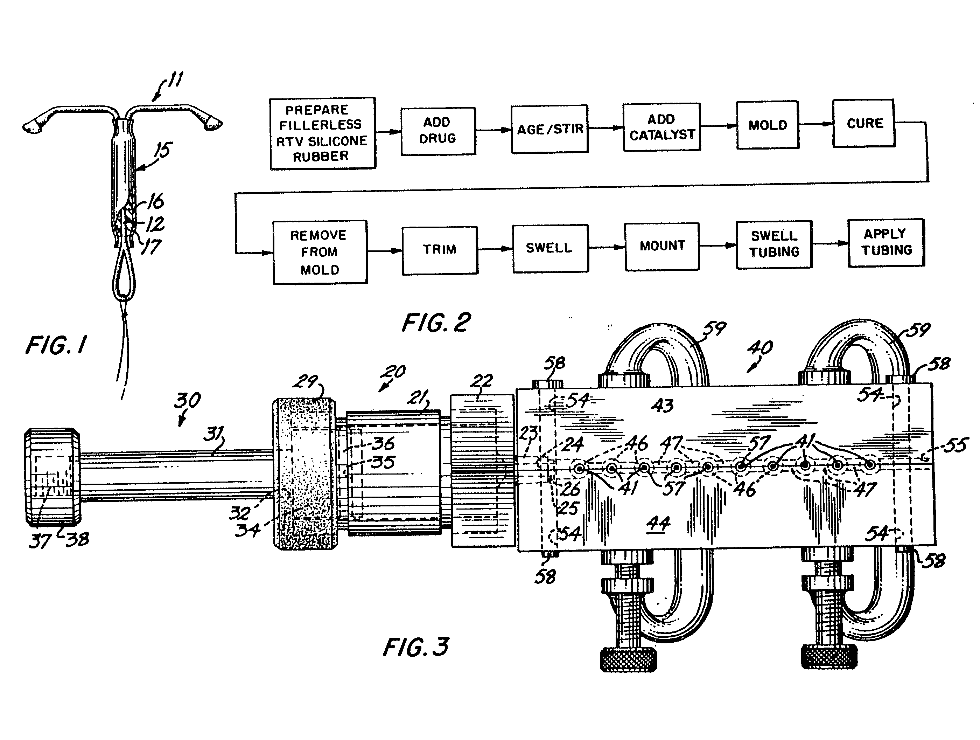

As illustrated in Figure 1, an existing IUD 11 includes a stem portion 12 that supports a drug-dispensing attachment 15. The attachment includes a sleeve 16 of a carrier or base material such as silicone rubber with a drug evenly distributed throughout. Overlying the sleeve 16, a length of drug permeable tubing 17 tightly fits about the sleeve.

The combination IUD and drug-dispensing attachment of Figure 1 is made by the method diagrammatically outlined in Figure 2. First, suitable, medically acceptable, silicone rubber is provided. Room temperature vulcanizing silicone rubber, which is easy to use and readily commercially available, can be prepared by centrifuging the silicone rubber until all filler material has been removed. For example, Medical Grade 382 Silastic (Trade Mark), a product of the Dow Corning Corporation, Midland, Michigan, has successfully been employed by centrifuging the material for several hours at 100,000 to 200,000 g.

The drug to be dispensed *in utero* by the attachment 15 is mixed with the silicone rubber. Micronized levonorgestrel was successfully mixed with prepared 382 Silastic (Trade Mark) by folding the powdered drug into the polymer a little at a time with stirring until a smooth paste of the two components was attained. To assure that the polymer had thoroughly "wet" the drug, the mixture was stirred several times a day for three or four days before use.

Next, the polymer-drug mixture is molded into the form of the sleeve 16 of Figure 1. To do this, a suitable catalyst is added to the drug-polymer mixture. The mixture is injected into a mold and allowed to cure until solid enough for removal without damaging the sleeves.

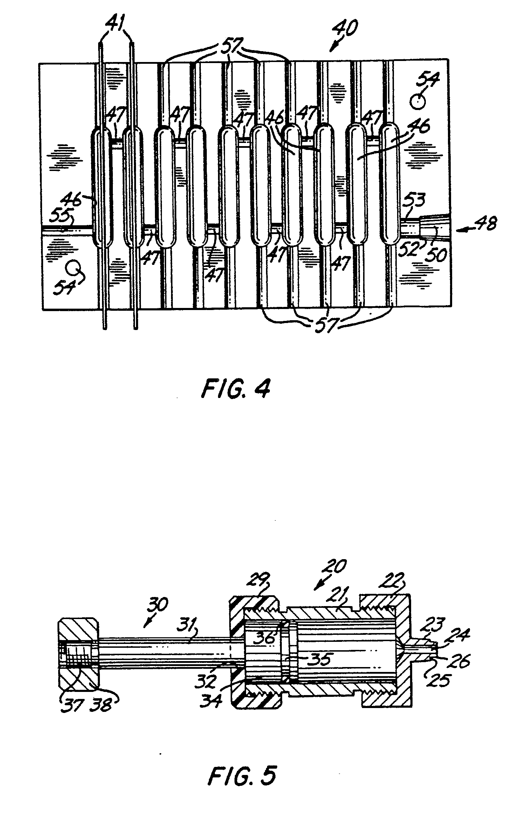

A special syringe 20, Figures 3 and 5, can be used to inject the viscous polymer and drug mixture into a multicavity mold 40, Figures 3 and 4. Both the syringe 20 and the mold 40 are discussed in greater detail below. In the successful example using 382 Silastic (Trade Mark) silicone rubber and levonorgestrel, Dow Corning Catalyst M, stannous octoate, was the catalyst. This was added and quickly stirred into the mixture of silicone rubber and drug. The syringe was loaded with the catalyzed viscous paste and forced into the mold, using a hydraulic jack to apply pressure. The mixture was allowed to cure for 45 minutes to 1 hour, or until the mixture had become solid enough to allow removal from the mold without damaging the sleeves. The cure time was conveniently judged by testing the hardness of the remnants of the mixture remaining on a Teflon (Trade Mark) sheet whereon the catalyst and mixture had been mixed.

After curing, the sleeves are removed from the mold. Interconnections formed by the passages in the mold are trimmed away by cutting with a scalpel or razor blade. The mold 40 of Figures 3 and 4 employs stainless steel tubes 41 as inserts that define a central opening 18 through the sleeves. Before mounting, the inserts are removed, again with care not to damage the fillerless silicone and drug sleeves. To remove the inserts, the sleeves can be swollen by immersing for a few minutes in a solvent chosen not to dissolve or otherwise adversely affect the silicone rubber or the intermixed drug. The 382 Silastic (Trade Mark) and levonorgestrel sleeves of the example were swollen in cyclohexane. Similarly, when expanded by solvent, the sleeves are easily mounted by slipping the sleeves onto the stems 12 of the extant IUDs 11.

To slow the rate of release of drug, and to impart greater structural integrity to the attachment, the section of drug permeable tubing 17 can be placed over the sleeve on the IUD. A commercially available silicone rubber tube can be swollen in a non-destructive solvent, and the tubing can be easily slipped onto the sleeve and allowed to shrink in place. In the example described above, Silastic (Trade Mark) Medical Grade Tubing 602—261 was swollen in cyclohexane for the purpose.

As shown in Figure 3 and mentioned briefly above, the apparatus for molding the drug-polymer mixture into the form of sleeves 16 includes the specially designed and interfitting syringe 20 and mold 40. Referring to Figure 5, the syringe includes a a hollow, cylindrical, stainless steel body 21. The outer surface of the body 21 is threaded at each end. It receives an interior-threaded stainless steel end cap 22 that has a tapered nipple 23. The nipple is centrally opened by a passage 24. Its outer taper terminates at a shoulder 25 from which projects a slight raised central tip 26.

At its other end, the syringe body 21 receives an internally threaded cap 29 of Delrin (Trade Mark), for example. A plunger 30 has a shaft 31 that passes through a closely fitting opening 32 in the cap 29. Within the body 21, the plunger 30 terminates in an enlarged head 34. A peripheral groove 35 seats a sealing O-ring 36 that bears against the interior of the body 21. Remote from the head 34, a reduced, threaded end 37 of the plunger 30 has an internally threaded stainless steel nut 38 secured thereon.

The mold 40 includes two plates 43 and 44 that combine to form the multicavity mold as illustrated in Figure 3. Figure 4 shows, in greater detail, the plate 44. Multiple recesses 46 in the face of the plate combine with like recesses 46 in the remaining plate 43 to form the multiple cavities of the mold. A series of grooves 47 forms, with aligned similar grooves 47 in the plate 43, connecting passages between the cavities 46, by which the polymer-drug mixture is conducted from one cavity 46 to the next during injection. Likewise, aligning halves of an injection opening are formed in the plates as shown at 48. The opening has a tapered bore 50, whose taper matches that of the syringe nipple 23. The tapered bore 50 ends in a shoulder 52, beyond which extends a passage 53 that opens into the first cavity 46. Matching alignment holes 54 through the plate facilitate aligning the plates and their cooperating recesses when the mold is closed. From the recess 46 that is farthest from the injection opening 48, a last passage 55 communicates with the exterior of the mold.

Coaxially with each of the recesses 46 and extending from each end thereof, a groove 57 leads from each recess 46 to the mold exterior. Again these are mirrored in the remaining mold plate 43. These grooves locate the inserts 41 that form the central openings in the drug release sleeves. In a preferred embodiment the inserts were stainless steel tubes, but of course they could be pins or other elongate members of a suitable material and of a width satisfactory to provide an opening through the sleeves that will tightly fit onto the stem of the platform.

Returning to Figure 3, the plates 43 and 44 are shown aligned by pins 58 extending through the alignment openings 54 in the upper and lower plates. The plates are clamped together by any convenient means. C-clamps 59 are shown in Figure 3, but it will be recognized that any clamping arrangement can be used, including for example bolting the plates together through aligned apertures. In the preferred embodiment the plates were formed of Delrin (Trade Mark). Other suitable materials will be apparent to those skilled in the art.

In use, the cavity within the syringe 20 is filled with the drug-polymer mixture after the catalyst has been added to the mixture. The syringe is closed, the nipple 23 is inserted into the injection opening 48, and pressure is applied to the nut 38 at the end of the syringe plunger 30 by, for example, a hydraulic jack. The matching tapers of the stainless steel nipple 23 on the syringe and in the Delrin (Trade Mark) injection opening bore 50 in the mold produce substantially leak-free mating of the two parts.

In the example discussed above, equal parts by weight of the polymer, Medical Grade 382 Silastic (Trade Mark), and the steroid levonorgestrel, were mixed as described, by folding the powdered drug into the polymer a little at a time with stirring to form a smooth paste. The repeated stirring over the course of several days followed. The mold 40 was provided with cavities 46 and inserts 41 having dimensions to give a sleeve length approximately 3/4″ (actually 18 mm), an outside diameter of about 1/8″ (3.175 mm), and an opening down the center of about 0.060″ (1.5 mm).

Six grams of the drug-polymer mixture was weighed onto a sheet of Teflon (Trade Mark) and 15 mg of the Dow Corning Catalyst M (stannous octoate) was quickly stirred into the mixture. The catalyst mixture was loaded into the special syringe 20. This was injected into the clamped-closed mold and allowed to cure for 45 minutes to 1 hour. The hardness of the remnants of the mixture remaining on the Teflon (Trade Mark) sheet, at that time, indicated sufficient curing.

When the mold was opened, the result was a "christmas tree" of sleeves interconnected with each other at alternate ends by the short sections of cured mixture formed in the passages 47 of the mold. The sleeves were separated by cutting the interconnections. The sleeves were swollen in cyclohexane and the tubular inserts 41 were removed. The ends of the sleeves were rounded to present a smooth surface to the endometrium of the uterus.

Using cyclohexane the sleeves were swollen, slipped onto the IUD stem and permitted to shrink thereon, again as described above. Lengths of the Silastic (Trade Mark) Medical Grade Tubing 602—261, sufficiently long to cover the length of the sleeve on the stem, were provided. This tubing had a 2.41 mm outside diameter and a 1.98 mm inside diameter. The wall thickness was thus 0.22 mm. As described above, the tubing was swollen in cyclohexane, placed over the sleeve on the stem, and allowed to dry and contract.

The drug release sleeve of the example was tested *in vitro*, and 20 to 25 mcg of levonorgestrel was delivered per day from the sleeve to the surrounding water. In one instance, *in vivo* testing indicated an average 10.5 mcg per day of the levonorgestrel delivered to the human uterus during a period of 287 days. This same device was then tested *in vitro* and released 20 mcg per day. The steroid was extracted and it was found that 94% of the original steroid had remained in the device.

In another *in vivo* test, a drug release device from the same lot as that just described, was *in utero* for 503 days. Analysis indicated that this device had released an average of 30 mcg per day during its residence in the human uterus.

The variation in extraction of the drug in the two *in vivo* studies was similar to variations observed in the extraction of levonorgestrel from other types of devices, such as subdermal implants. This is believed to result from the heterogeneous characteristics of the individuals, rather than from any variation in the characteristics of the drug release devices. Variations in the extracting fluids produced in the uterus will vary the rate of extraction of the drug. Levonorgestrel has an atrophic affect, tending to dry the endometrium. While this can slow the release of the drug from the device, this also contributes to the contraceptive effect of the drug, helping to prevent embryo implantation.

Two other sizes of sleeves have been tested, as well. Smaller sleeves, 13 mm long with 3.34 mm diameters, had a total surface area of 136 mm² each. These were designed to deliver 23 mcg per day of the levonorgestrel. The outer membrane covering provided by the tubing was as described above. Ten of the sleeves were recovered after a mean residence time *in utero* of 281 days (actual residence times range from 98 to 500 days). The amount of levonorgestrel delivered was 22 ± 7 mcg per day (mean ± standard deviation). Larger sleeves, 18 mm long with 3.34 mm diameters, had total surface areas of 189 mm² each, were enclosed in the aforementioned tubing, and gave comparably good results. These larger sleeves were designed to deliver 32 mcg per day of the levonorgestrel. Fourteen were recovered after a mean residence time *in utero* of 314 days (range 84 to 482 days). The amount of levonorgestrel delivered was 30 ± 9 mcg per day (mean ± standard deviation). At the mean rates of release, the attachments just described would be expected to deliver the drug for more than four years.

Dosage can be controlled in several ways. Either or both of the sleeve diameter and length can be adjusted to alter the sleeve surface area. With or without the outer tubing layer, the rate of delivery of drug is a direct function of the total surface area of the sleeve. Reducing the surface area by half, then, will halve the rate of delivery, assuming all other parameters remain the same. Without the outer tubing, the rate of delivery is initially very rapid, giving a "bolus" effect, then declines rather quickly. When the outer tubing is used, the release rate is relatively constant. It is controlled by both the surface area of the sleeve and the wall thickness of the drug permeable tube. For the sleeve of given dimensions, modification of the wall thickness of the silicone rubber tube as described above modifies the release rate, but not in direct porportion to thickness. A doubling of the wall thickness of the tube results in a release rate of about 60% of a single thickness, rather than 50% thereof.

Varying the sleeve and tubing dimensions, provides control of the life as well as the dosage of the sleeves. Long term contraception, as much as seven years, is currently envisioned. Increasing the diameter of the sleeve would allow more drug to be put into the device, and putting on a thicker tube would lower the release rate. Although increasing the diameter would increase the surface area, the thicker tubing would decrease the rate of release per unit of surface. Release rates similar to those described above could thus be achieved plus an increase in effective life.

Placement of the combined device in the uterus is no more difficult than placement of the platform itself. In fact, placement techniques identical to those used with the ordinary, unmedicated IUD's have successfully been employed. No specially formed IUD is necessary. The Nova-T has been preferred as the base platform on which the drug release sleeve is mounted. It is easy to handle, assures that the sleeve will stay in place, and is easily inserted.

In addition to the dosage factors discussed above, the diameter of the sleeve 16 is chosen in connection with the inside diameter of the inserted tube used in placement of the IUD. In the case of the Nova-T-platform, the arms fold up and can fit into a very narrow inserter tube. The sleeve diameter, then governs the size of the inserter tube. As can be appreciated from Figure 1, the sleeve diameter is such that little or no increase is necessary in the diameter of the inserter tube and no difficulty in placement of the IUD and sleeve results.

The combination IUD platform and drug release attachment made by the method described above gives good contraceptive benefit. It acts as a known, unmedicated IUD, and as a controlled dosage, directly administering contraceptive drug dispensing device. Another advantage appearing to result from the use of the sleeve described above is a decrease in menstrual blood loss. Drug release intrauterine devices currently on the market appear to give this effect, as well, but with those, higher extrauterine pregnancy rates have been observed.

Drugs delivered with devices of the kind made by the method described above may be any of those useful for treatment of any condition of the uterus or endrometrium thereof, as well as steroids useful in providing contraceptive effects, whether of local or of systemic activity. Although only the room temperature vulcanizing silicone rubber as been used so far in forming the sleeves, many other polymer and drug mixtures will prove useful in forming these. Likewise, only silicone rubber tubing as described above has been used to control the release rate and impart greater structural integrity, but the other membrane, may, as well, be composed of other biocompatible polymers that may be applied in a variety of ways, such as by dip-coating, heatsealing, spraying, or otherwise.

For the construction of the mold 40, Food-Grade Delrin (Trade Mark) was chosen, but a suitable mold could be formed of, for example, carbon steel, stainless steel, brass, aluminum, polycarbonates, polyacetates, lucite, Teflon (Trade Mark), polyethylene, polypropylene, polysulfone, and other materials of known, suitable strength and easy of manufacture. The same is true as to choice of materials for both the inserts 41 for the mold and the parts of syringe 20.

Any of a number of solvents can be used to swell the silicone rubber and drug sleeve and the silicone rubber tubing. Cyclohexane was chosen for its quickness in swelling these members, the greater extent to which it swells them, and for its safety. However, any of the hexanes, straight-chain parafins, and normal hydrocarbons that are fully saturated should suffice. Again, the solvent should dissolve neither the steroid nor the silicone rubber. In this relation, ether, toluene or chloroform would surely be unsatisfactory.

Although particular preferred embodiments of the method of making the IUD platform and attachment are described and illustrated herein, many modifications thereof will be apparent to those skilled in the art, without departure from the scope of the invention as set forth in the appended claims.

## Claims

1. A method of making a combination of intrauterine device and drug release element, comprising providing a formable silicone rubber as a carrier material and providing a non-medicated and relatively stable platform (11) for intrauterine placement, characterized by mixing together a drug for in utero administration and the formable carrier material, the rubber of which being a non-filler-containing silicone rubber, forming the mixture of drug and the carrier material into an attachment (15) adapted to fit onto the platform (11), and mounting the attachment (15) on the platform (11) for controlled release of the drug in utero, said mounting being effected by expanding the attachment (15) by immersion in a solvent suitable for swelling the rubber and in which the rubber and the drug are substantially insoluble, and then shrinking the expanded attachment (15) about a portion (12) of the platform (11).

2. The method according to claim 1, characterized in that the step of providing a non-filler-containing formable silicone rubber further comprises providing a formable silicone rubber containing a filler and removing the filler from the silicone rubber prior to mixing it with the drug to enhance in utero drug release by the attachment (15).

3. A method according to claim 1 or 2 further comprising the step of placing a drug permeable covering (17) over the attachment (15) whereby the administered drug is controlled as a function of the thickness of the covering (17).

4. The method according to claim 1 or 2, characterized in that the step of forming further includes providing a pair of plates (43, 44) with aligned multiple recesses (46) therein to define the multiple cavities of the mold, securing the plates together with the recesses in alignment, locating elongate central opening forming members (41) in the cavities defined by the recesses to establish central axial openings through the molded sleeves, separating the plates after the carrier and drug mixture has set, and trimming undesired portions from the sleeves prior to application to the intrauterine platforms.

5. The method according to claim 1 or 2, characterized in that the step of forming includes defining an opening through the attachment, and the step of mounting includes temporarily expanding the attachment with the solvent and placing a stem (12) of the intrauterine platform through the opening for subsequent shrinkage of the attachment (15) about the stem (12).

6. The method according to claim 1 or 2, characterized in that the solvent is cyclohexane.

7. The method according to claim 2, characterized in that the step of removing the filler from the silicone rubber includes centrifuging the silicone rubber prior to mixing the drug and silicone rubber.

8. The method according to claim 1 or 2, characterized in that the step of forming includes loading a syringe (20) with a quantity of the mixture of carrier material and drugs, and injecting the quantity into a multicavity mold (48) to mold attachments in the form of sleeves dimensioned to fit on a plurality of the pre-existing intrauterine platforms (11).

9. The method according to claim 2, characterized in that the filler removed from the silicone rubber is a reinforcing material for adding structural integrity to the formed silicone rubber, and the method further includes the step of placing an outer drug permeable covering in tightly fitting and reinforcing relation over the attachment.

10. The method according to claim 9, characterized in that the step of placing an outer covering includes shrinking a length of preformed tubing tightly about the attachment (15) on the intrauterine platform (11).

11. The method according to claim 3, characterized in that the step of placing the covering (17) comprises temporarily expanding a length of drug permeable tubing, and placing the tubing over the attachment for subsequent shrinkage of the tubing about the attachment.

## Revendications

1. Procédé de réalisation d'une combinaison de dispositif intra-utérin et d'elément de libération d'un médicament comprenant la prévision d'un caoutchouc silicone pouvant être façonné comme un matériau de support et la prévision d'une plateforme non médicamentée et relativement stable (11) pour pose intra-utérine, caractérisé en ce qu'on mélange ensemble un médicament pour administration in utéro et le matériau de support pouvant être façonné dont le caoutchouc est un caoutchouc silicone ne contenant pas de charge, on façonne le mélange médicamenteux et le matériau de support en un accessoire (15) propre à être adapté sur la plateforme (11), et l'on monte l'accessoire (15) sur la plateforme (11) en vue de la libération contrôlée du médicament in utéro, ce montage étant effectué en dilatant l'accessoire (15) par immersion dans un solvant propre à faire gonfler le caoutchouc, dans lequel le caoutchouc et le médicament sont sensiblement insolubles, puis en provoquant le retrait de l'accessoire dilaté (15) autour d'une partie (12) de la plateforme (11).

2. Procédé selon la revendication 1, caractérisé en ce que la phase de prévision d'un caoutchouc silicone pouvant être façonné ne contenant pas de charge comprend encore la prévision d'un caoutchouc silicone pouvant être façonné, contenant une charge et la suppression de la charge contenue dans le caoutchouc silicone avant de le mélanger avec le médicament pour favoriser la libération in utéro du remède par l'accessoire (15).

3. Procédé selon la revendication 1 ou 2 comprenant outre la phase de mise en place d'une enveloppe perméable au médicament (17) sur l'accessoire (15), de sorte que le médicament administré soit contrôlé en fonction de l'épaisseur de l'enveloppe (17).

4. Procédé selon la revendication 1 ou 2, caractérisé en ce que la phase de façonnage comporte en outre la prévision de deux plaques (43, 44) présentant des évidements multiples alignés (46) destinés à définir les cavités multiples du moule, la fixation des plaques ensemble avec alignement des évidements, la mise en place d'organes de mise en forme d'ouvertures centrales oblongues (41) dans les cavités définies par les évidements pour créer des ouvertures axiales centrales dans les manchons moulés, la séparation des plaques après la prise du mélange de support et de médicament et l'ébavurage des parties indésirables des manchons avant pose sur les plateformes intra-utérines.

5. Procédé selon la revendication 1 ou 2, caractérisé en ce que la phase de façonnage comporte la création d'une ouverture à travers l'accessoire, et la phase de montage comprend la dilatation temporaire de l'accessoire par le solvant et l'insertion d'une tige (12) de la plateforme intra-utérine à travers l'ouverture en vue du retrait ultérieur de l'accessoire (15) autour de la tige (12).

6. Procédé selon la revendication 1 ou 2, caractérisé en ce que le solvant est du cyclohexane.

7. Procédé selon la revendication 2, caractérisé en ce que la phase d'élimination de la charge du caoutchouc silicone comporte le centrifugeage du caoutchouc silicone avant mélangeage du médicament et du caoutchouc silicone.

8. Procédé selon la revendication 1 ou 2, caractérisé en ce que l'opération de façonnage comprend le remplissage dans une seringue d'une certaine quantité de mélange de matériau de support et de médicament et l'injection de cette quantité dans un moule (48) à plusieurs cavités pour le moulage d'accessoires en forme de manchons, dimensionnés de façon à s'emboîter sur une série des plateformes intra-utérines préexistantes (11).

9. Procédé selon la revendication 2, caractérisé en ce que la charge éliminée du caoutchouc silicone est un matériau de renfort pourt améliorer l'intégrité structurelle du caoutchouc silicone façonné, et que le procédé comporte encore la phase de mise en place d'une enveloppe extérieure perméable au médicament, serrant étroitement l'accessoire et le renforçant.

10. Procédé selon la revendication 9, caractérisé en ce que l'opération de mise en place d'une enveloppe extérieure comprend le retrait d'une longueur de tube préfaçonné entourant étroitement l'accessoire (15) sur la plateforme intra-utérine (11).

11. Procédé selon la revendication 3, caractérisé en ce que la phase de mise en place de l'enveloppe (17) comprend la dilatation temporaire d'une longueur de tube perméable au médicament et la pose du tube sur l'accessoire en vue du retrait ultérieur du tube sur l'accessoire.

**Patentansprüche**

1. Verfahren zur Herstellung einer Kombination aus einem Intrauterinpessar und einem Arzneistoff-Freisetzungselement, bestehend darin, daß man formbaren Silikonkautschuk als Trägermaterial und ein nicht-medizinisches und relativ stabiles Formteil (11) zur intrauterinen Plazierung verwendet, dadurch gekennzeichnet, daß man einen Arzneistoff für in utero-Verabreichung und das formbare Trägermaterial, dessen Kautschuk ein keinen Füllstoff enthaltender Silikonkautschuk ist, zusammengibt, das Gemisch aus dem Arzneistoff und dem Trägermaterial zu einem Befestigungsteil (15) formt, das auf das Formteil (11) paßt, und das Befestigungsteil (15) zur kontrollierten Freisetzung des Arzneistoffes im Uterus auf dem Formteil (11) anbringt, wobei das Anbringen dadurch erreicht wird, daß sich das Befestigungsteil (15) beim Eintauchen in ein Lösungsmittel, das zum Quellen des Kautschuks geeignet ist und in dem der Kautschuk und der Arzneistoff praktisch unlöslich sind ausdehnt und anschließend das gedehnte Befestigungsteil (15) um einen Teil (12) des Formteils (11) schrumpfen läßt.

2. Verfahren nach Anspruch 1, dadurch gekennzeichnet, daß der Schritt, in dem ein keinen Füllstoff enthaltender formbarer Silikonkautschuk verwendet wird, zusätzlich dadurch charakterisiert ist, daß man einen formbaren Silikonkautschuk mit einem Füllstoff verwendet und den Füllstoff aus dem Silikonkautschuk vor dem Vermischen mit dem Arzneistoff entfernt, um die Arzneistoff-Freisetzung im Uterus durch das Befestigungsteil (15) zu verstärken.

3. Verfahren nach Anspruch 1 oder 2, einen weiteren Schritt enthaltend, der darin besteht, daß man das Befestigungsteil (15) mit einem für einen Arzneistoff durchlässigen Überzug (17) versieht, wobei der verabreichte Arzneistoff durch die Dicke des Überzugs (17) kontrolliert wird.

4. Verfahren nach Anspruch 1 oder 2, dadurch gekennzeichnet, daß der Formungsschritt zusätzlich dadurch charakterisiert ist, daß man ein Plattenpaar (43, 44) mit zueinander ausgerichteten zahlreichen Aussparungen (46) darin, die die zahlreichen Vertiefungen des Formstückes festlegen, verwendet; die Platten zusammenpreßt, wobei die Aussparungen zueinander ausgerichtet sind; längliche, eine zentrale Öffnung bildende Bauelemente (41) in den durch die Aussparungen festgelegten Vertiefungen anordnet, um zentrale axiale Öffnungen durch die geformten Hülsen zu erhalten; die Platten trennt, nachdem das Gemisch aus Träger und Arzneistoff erstarrt ist; und unerwünschte Teile von den Hülsen vor dem Aufbringen auf die intrauterinen Formteile abschneidet.

5. Verfahren nach Anspruch 1 oder 2, dadurch gekennzeichnet, daß der Formungsschritt die Ausbildung einer Öffnung durch das Befestigungsteil enthält, und daß der Anbringungsschritt ein zeitweiliges Ausdehnen des Befestigungsteils mit Hilfe des Lösungsmittels und das Einbringen eines Schafts (12) des intrauterinen Formteils durch die Öffnung für das nachfolgende Schrumpfen des Befestigungsteils (15) um den Schaft (12), umfaßt.

6. Verfahren nach Anspruch 1 oder 2, dadurch gekennzeichnet, daß das Lösungsmittel Cyclohexan ist.

7. Verfahren nach Anspruch 2, dadurch gekennzeichnet, daß der Schritt zur Entfernung des Füllstoffes aus dem Silikonkautschuk die Zentrifugation des Silikonkautschuks vor dem Vermischen des Arzneistoffs und des Silikonkautschuks enthält.

8. Verfahren nach Anspruch 1 oder 2, dadurch gekennzeichnet, daß der Formungsschritt das Füllen einer Spritze (20) mit einer Menge des Gemisches aus Trägerstoff und Arzneistoffen und das Injizieren der Menge in eine viele Vertiefungen enthaltende Form (48) enthält, um Befestigungsteile in der Form von Hülsen zu formen, die so gebaut sind, daß sie auf eine Vielzahl der Schon vorhandenen intrauterinen Formteile (11) passen.

9. Verfahren nach Anspruch 2, dadurch gekennzeichnet, daß der aus dem Silikonkautschuk entfernte Füllstoff ein Verstärkungsmaterial ist, das dem gebildeten Silikonkautschuk strukturelle Integrität verleiht, und daß das Verfahren einen weiteren Schritt enthält, in dem ein äußerer für einen Arzneistoff durchlässiger Überzug eng anliegend und zur Verstärkung auf dem Befestigungsteil aufgebracht wird.

10. Verfahren nach Anspruch 9, dadurch gekennzeichnet, daß das Aufbringen eines äußeren Überzuges das Schrumpfenlassen eines Abschnittes eines vorgeformten Schlauches, so daß er dicht an dem Befestigungsteil (15) auf dem intrauterinen Formteil (11) anliegt, enthält.

11. Verfahren nach Anspruch 3, dadurch gekennzeichnet, daß der Schritt des Aufbringes des Überzuges (17) das zeitweilige Dehnen eines Abschnittes des für den Arzneistoff durchlässigen Schlauchs und das Überziehen des Schlauchs über das Befestigungsteil zum nachfolgenden Schrumpfen des Schlauches um das Befestigungsteil enthält.

FIG. 1

PREPARE FILLERLESS RTV SILICONE RUBBER → ADD DRUG → AGE/STIR → ADD CATALYST → MOLD → CURE

REMOVE FROM MOLD → TRIM → SWELL → MOUNT → SWELL TUBING → APPLY TUBING

FIG. 2

FIG. 3

0 082 894

0 082 894

FIG. 4

FIG. 5